# EUROPÄISCHE PATENTSCHRIFT

(11) **EP 2 185 130 B1**
(45) Veröffentlichungstag und Bekanntmachung des Hinweises auf die Patenterteilung: **12.09.2012**
(21) Anmeldenummer: 08785371.9
(22) Anmeldetag: 06.08.2008
(51) Int. Cl.: A61K 9/16, A61K 9/20, A61K 31/445

(54) **VERFAHREN ZUR HERSTELLUNG EINER DONEPEZILHYDROCHLORID IN AMORPHER FORM ENTHALTENDEN TABLETTE**
PROCESS FOR PREPARING A TABLET CONTAINING DONEPEZIL HYDROCHLORIDE IN AMORPHOUS FORM
PROCÉDÉ POUR LA PRÉPARATION D'UN COMPRIMÉ CONTENANT DU CHLORHYDRATE DE DONÉPÉZIL SOUS FORME AMORPHE

(30) Priorität: 11.08.2007 DE 102007037932
(43) Veröffentlichungstag der Anmeldung: 19.05.2010
(73) Patentinhaber: Zhejiang Huahai Pharmaceutical Co., Ltd., Zhejiang 317-024 (CN); Alfred E. Tiefenbacher GmbH & Co. KG, 22767 Hamburg (DE)
(72) Erfinder: HONGXI, Wang, Shanghai, 0201114 (CN); JUNQING, Peng, Zhejiang (CN); GONGYUN, Hu, Zhejiang (CN)
(74) Vertreter: von Seebach, Malte
(86) Internationale Anmeldenummer: PCT/EP2008/006446
(87) Internationale Veröffentlichungsnummer: WO 2009/021656

(56) Entgegenhaltungen:
- EP-A- 1 378 238
- WO-A-2005/105054
- WO-A-2006/045512
- DE-A1-102005 060 377

## Beschreibung

Die vorliegende Erfindung betrifft ein Verfahren zur Herstellung einer Tablette zur Behandlung der Demenz oder der Alzheimerkrankheit, die das Hydrochloridsalz von Donepezil in amorpher Form enthält. Donepezilhydrochlorid ist Wirkstoff für die Behandlung der Demenz und Alzheimerkrankheit. Donepezilhydrochlorid ist ein Acetylcholinesterase-Hemmer, der unter dem Namen Aricept^{®} in Form von 5 mg- oder 10 mg-Filmtabletten vermarktet wird.

Das europäische Patent EP 0 296 560 beschreibt im Beispiel 4 erstmalig die Herstellung des Hydrochloridsalzes von Donepezil. Donepezilhydrochlorid zeigt einen ausgeprägten Polymorphismus. So beschreibt WO 97/46527 fünf verschiedene kristalline Formen von Donepezilhydrochlorid, nämlich die polymorphen Formen I-V. Die Polymorphe I und IV sind kristalline Hydrate und weisen einen Wassergehalt von ca. 5 %-6 % bzw. ca. 11 %-12 % auf. Die Polymorphe II, III und V sind dagegen keine Hydrate. WO 97/46527 beschreibt in den Beispielen, dass die polymorphe Form I von Donepezilhydrochlorid durch Trocknen bei vermindertem Druck in seine polymorphe Form II oder durch Trocknen bei Normaldruck in seine polymorphe Form III umgewandelt werden kann. Außerdem werden in den Beispielen die Umwandlung von amorphem Donepezilhydrochlorid in die polymorphe Form II oder IV, die Umwandlung der Form II in die Form IV und die Umwandlung der Form IV in die Form V beschrieben.

WO 2004/087660 offenbart ein Verfahren zur Herstellung von amorphem Donepezilhydrochlorid, wobei Donepezilhydrochlorid in einem Gemisch aus einem alkoholischen Lösungsmittel und einem chlorierten Lösungsmittel wie Dichlormethan oder Chloroform gelöst und anschließend die Lösungsmittel z.B. durch Gefriertrocknen, Destillation unter vermindertem Druck, Sprühtrocknung usw. entfernt wird.

WO 2004/092137 beschreibt die kristallinen Formen H1 und H2, sowie ein Monohydrat und Sesquihydrat von Donepezilhydroohlorid.

DE 10 2005 060 377 beschreibt Verfahren zur Herstellung einer Tablette, die Doneperilhydrochlorid in der kristallinen Form I enthält, wobei Doneperilhydrochlorid in der kristallinen Form I mit Hilfontoffen gemischt und einer Direktverpressung unterzogen wird.

Im Falle einer ausgeprägten Polymorphie ist es wichtig, dass in der pharmazeutischen Zusammensetzung, mit welcher Donepozilhydrochlorid dem Patienten verabreicht wird der Wirkstoff in ein und derselben kristallinen Form, auch nach Lagerung, enthalten ist, um eine gleichbleibende therapeutische Aktivität des Wirkstoffes zu gewährleisten. Es ist bekannt, dass verschiedene kristalline Formen ein und desselben Wirkstoffes sich zum Beispiel in der Löslichkeit und der Auflösungsgeschwindigkeit unterscheiden, was die Absorptionsrate und die Bioverfügbarkeit des Wirkstoffes entscheidend beeinflussen kann, Für das Donepezilhydrochlorid ist das Stabilitätserfordernis nicht leicht zu erfüllen, da sich seine kristallinen polymorphen Formen, wie z.B. in WO 97/46527 offenbart, leicht ineinander umwandeln lassen, Des Weiteren können bereits geringfügige Modifikationen ein und desselben Kristallisationsverfahrans unterschiedliche polymorphe Formen von Donepezilhydrochlorid ergeben.

Die Verwendung eines amorphen Materials stellt eine gute Lösung für die vorstehend beschriebenen Probleme bei einem Wirkstoff mit ausgeprägtem Polymorphismus dar. Nichtkristallines oder amorphes Donepezilhydrochlorid weist sowohl eine gute Löslichkeit als auch eine schnelle Auflösungsrate auf, so dass eine gute Bioverfügbarkeit gewährleistet ist.

So beschreibt EP-A-1 378 238 A pharmazeutische Zusammensetzungen, die ein Donepezilhydrochlorid in amorpher Form enthalten. Tabletten, die Donepezilhydrochlorid in amorpher Form enthalten, wurden durch Nassgranulierung erhalten, wobei Donepezilhydrochlorid zunächst in Wasser gelöst und die erhaltene Lösung mit einem festen Gemisch aus Trägem und Hilfsstoffen granuliert wurde. Nassgranulierung durch Lösen des Donepezilhydrochlorids in der Granulierflüssigkeit führt somit zu einem Granulat, in welchem der Wirkstoff in amorpher Form vorliegt.

WO 2004/000317 offenbart die Herstellung von amorphes Donepezilhydrochlorid enthaltenden pharmazeutischen Zusammensetzungen, das dadurch gekennzeichnet ist, dass das Donepezilhydrochlorid in situ durch Lösen oder Dispergieren von Donepezil als freie Base in einem pharmazeutisch verträglichen Lösungsmittel und einer Salzsäurelösung hergestellt und durch Zugabe eines Kristallisationsinhibitors stabilisiert wird. In einer alternativen Ausführungsform kann die in situ Herstellung eines amorphen Donepezilhydrochlorids in Gegenwart eines Kristallisationsinhibitors durchgeführt werden. Als bevorzugte Kristallisationsinhibitoren werden Cellulosederivate und Polyvinylpyrrolidon, sowie Derivate davon, genannt.

WO 2004/071486 offenbart pharmazeutische Zusammensetzungen, die Donepezilhydrochlorid in amorpher Form enthalten. Es wird ausgeführt, dass amorphes Donepezilhydrochlorid, das durch Lyophilisierung einer wässerigen Lösung hergestellt wurde, extrem hygroskopisch ist und dazu neigt, Wasser und Feuchtigkeit aus der Luft zu absorbieren, was die Verarbeitbarkeit des Materials erheblich negativ beeinflusst, insbesondere hinsichtlich seiner Fließeigenschaften und physikalischen Stabilität (z.B. kann eine Rekristallisation auftreten). WO 2004/071486 beschreibt des weiteren, dass die Durchführung einer Lyophilisierung einer das Hydrochloridsalz von Donepezil enthaltenden wässerigen Lösung zu einem nicht hygroskopischen, chemisch wie auch physikalisch stabilen Produkt führt, wenn die Lyophilisierung in Gegenwart eines inaktiven Bestandteils durchgeführt wird. Als besonders geeignete inaktive Bestandteile werden Lactose, Polyvinylpyrrolidon und Polyethylenglycol, sowie Gemische davon, angegeben.

WO 2005/065645 offenbart pharmazeutische Zusammensetzungen, die Donepezil oder ein pharmazeutische verträgliches Salz davon in amorpher Form enthalten, wobei durch Zusatz eines pharmazeutisch verträglichen polymeren Trägers die amorphe Form des Wirkstoffes in der Formulierung stabilisiert und beibehalten wird. Als geeignete polymere Träger werden zum Beispiel Hydroxypropylcellulose, Methylcellulose, Carboxymethylcellulose, Polyvinylalkohol, Polypropylen, Polyvinylchlorid, Polyvinylacetat, Polyethylenglycol, usw. genannt. Ein besonders bevorzugter polymerer Träger ist Polyvinylpyrrolidon mit einem Molekulargewicht von etwa 2500 bis etwa 3,0 Mio., vorzugsweise etwa 10.000 bis etwa 450.000. Die offenbarten Zusammensetzungen können hergestellt werden, indem das Donepezil oder ein pharmazeutisch verträgliches Salz davon, der polymere Träger und ein Lösungsmittel wie Wasser miteinander kombiniert werden, vorzugsweise so, dass eine Lösung erhalten wird, so dass nach Entfernen des Lösungsmittels eine feste Dispersion des Wirkstoffes in dem polymeren Träger vorliegt.

WO 2006/063025 offenbart physikalisch stabile pharmazeutische Zusammensetzungen, die Donepezil oder pharmazeutisch verträgliche Salze davon in amorpher Form enthalten, wobei physikalische Stabilität bedeutet, dass der amorphe Wirkstoff in der Zusammensetzung auch nach Lagerung, keiner Rekristallisierung unterliegt. Des Weiteren wird die Herstellung eines amorphen Donepezilhydrochlorids, insbesondere in Form eines Pulvers beschrieben, das die Umwandlung eines oder mehrerer der bekannten kristallinen Polymorphe von Donepezilhydrochlorid durch Lösen des Salzes in einem organischen Lösungsmittel, wie zum Beispiel in Methanol und/oder Dichlormethan, und Kontaktieren der erhaltenen Lösung mit einem Antilösungsmittel in einem super kritischen oder beinahe super kritischen Zustand, wie zum Beispiel superkritisches CO₂, umfasst, wodurch das Donepezilhydrochlorid in amorpher Form und als Teilchen aus der Lösung extrahiert wird. Außerdem wird ein Verfahren zur Herstellung eines stabilen amorphen Donepezilhydrochlorids in Teilchenform offenbart, in dem ein oder mehrere kristalline Polymorphe von Donepezilhydrochlorid in Gegenwart eines stabilisierenden Hilfsstoffs einem Sprühtrocknungsverfahren unterzogen werden.

Die im Stand der Technik bekannten Verfahren zur Herstellung von pharmazeutischen Zusammensetzungen, die Donepezilhydrochlorid in amorpher Form enthalten, verwenden amorphes Donepezilhydrochlorid als Ausgangsmaterial, entweder in nicht stabilisierter Form oder, durch Zusatz entsprechender Hilfsstoffe bei der in situ Herstellung, Lyophilisierung, Gefriertrocknung, Sprühtrocknung usw., in stabilisierter Form.

Dagegen offenbart WO 2006/045512 eine stabile pharmazeutische Zusammensetzung, die ein kristallines Donepezilhydrochlorid als Hydrat enthält und die einen Wassergehalt von 3-30 Gew.-% (Karl Fischer) aufweist. WO 2006/045512 offenbart, dass, wenn ein Hydrochloridsalz von Donepezil in einer kristallinen und hydratisierten Form, d.h. ein kristallines Hydrate als Ausgangsmaterial für die Herstellung von pharmazeutischen Zusammensetzungen verwendet wird, aufgrund der Stabilisierung als Hydrat keine Umwandlung des kristallinen Ausgangsmaterials während der Verarbeitung zu einer pharmazeutischen Zusammensetzung, z.B. nach einer Feuchtgranulierung, zu beobachten ist. Dieses Phänomen wird auf die erhöhte Stabilität der kristallinen Hydrate von Donepezilhydrochlorid zurückgeführt, wie es z.B. bei den polymorphen Formen I und IV auftritt. So wird die polymorphe Form I von Donepezilhydrochlorid als ein Monohydrat mit einem theoretischem Wassergehalt von ca. 4,1 Gew.% (Karl Fischer) bezeichnet. Außerdem wird beschrieben, dass, zusätzlich zu der erhöhten Stabilität der kristallinen und hydratisierten Hydrochloridsalze von Donepezil, die Umwandlung der kristallinen Hydrate in andere kristalline Formen von Donepezilhydrochlorid, wie z.B. in die dehydratisierten Formen durch Einstellung des Wassergehaltes der eingesetzten Hilfsstoffe vermieden werden kann. So sollte in einer bevorzugten Ausführungsform die Differenz des Wassergehaltes von dem eingesetzten kristallinen Hydrat von Donepezilhydrochlorid zu den jeweils anderen Hilfsstoffen weniger als 4 Gew.%, vorzugsweise weniger als 3 Gew.-% und am meisten bevorzugt weniger als 2 Gew.-% (Karl Fischer) betragen. Durch diese kleinen Differenzen in den Wassergehalten soll eine Wanderung des Kristallwassers aus dem eingesetzten Wirkstoff zu den übrigen Hilfsstoffen oder umgekehrt verhindert werden.

Angesichts des vorstehend beschriebenen Standes der Technik war es deshalb eine Aufgabe der vorliegenden Erfindung, ein Verfahren zur Herstellung einer das Hydrochloridsalz von Donepezil in amorpher Form enthaltenden Tablette bereitzustellen, mit dem die im Stand der Technik bekannten Schwierigkeiten bei der Verarbeitung des Hydrochloridsalzes von Donepezil in amorpher Form, bedingt z.B. durch seine Hygroskopie, niedrige Bulkdichte, schlechtes Fließverhalten und mangelhafte physikalische Stabilität, minimiert oder gar vermieden werden.

Diese Aufgabe wird durch das in den Ansprüchen angegebene Verfahren gelöst.

Es wurde gefunden, dass beim Verpressen einer Tablettenmasse umfassend ein Granulat, wobei das Granulat ein Hydrochloridsalz von Donepezil in einer kristallinen und dehydratisierten Form enthalt, eine Tablette erhalten werden kann, die wiederum das Hydrochloridsalz von Donepezil in amorpher Form enthält. Das erfindungsgemäße Verfahren ist außerdem dadurch gekennzeichnet, dass das kristalline, dehydratisierte hydrochloridsalz von Donepezil einen Restmassergehalt von 3-7 Gew.-% aufweist und das Granulat durch Feuchtgranulierung erhalten wird. Bei den kristallinen Hydrochloridsalzen von Donepezil in einer dehydratisierten Form handelt es sich um diejenigen kristallinen Hydrochloridsalze von Donepezil, die durch Trocknen, d.h. Dehydratisieren der kristallinen und hydratisierten Hydrochloridsalze von Donepezil zu erhalten sind. Als Beispiele eines Hydrochloridsalzes von Donepezil in einer kristallinen und dehydratisierten Form seien die polymorphen Formen II, III und V genannt, die z.B. aus den kristallinen Hydrate von Donepezilhydrochlorid, so zB. aus der polymorphen Form I gemäß der Beispiele 24-27 und 56 oder aus dem Polymorph IV gemäß Beispiel 109 aus WO 97/46527 durch Trocknen zu erhalten sind. Die kristallinen und dehydratisierten Hydrochloridsalze von Donepezil weisen in der Regel einen Restwassergehalt auf, so kann z.B. in Abhängigkeit von dem Herstellungsverfahren, den Lagerungsbedingungen oder der Umgebungsfeuchtigkeit "freies", also nicht im Kristall als Kristallwasser gebundes, Wasser an dem kristallinen und dehydratisierten Material adsorbiert sein, Demgemäß schwanken die in den Beispielen von WO 97/46527genannten Wassergehalte für das Polymorph II von 0,19 Gew.%-2,33 Gew.-%, für das Polymorph III von 0,03 Gew.-%-0,65 Gew.-% und für das Polymorph V wird lediglich ein Wassergehalt von 0,28 Gew.-% genannt, da nur ein Beispiel für die Herstellung des Polymorphs V angegeben wurde. Dem Fachmann ist natürlich bekannt, dass bei der Verarbeitung des dehydratisierten Materials (z.B. zu Tabletten) weit höhere Restwassergehalte (oder "Restfeuchten") von z.B. 3-7 Gew.-% oder 4-7 Gew.-% auftreten können, z.B. durch Adsorption von Wasser aus der Umgebung bei der Lagerung.

Die nach dem erfindungsgemäßen Verfahren hergestellte Tablette enthält das Hydrochloridsalz von Donepezil in amorpher Form. Diese Tablette weist üblicherweise Restwassergehalte von 2-15 Gew.-%, vorzugsweise 5-10 Gew.-% und am meisten bevorzugt 7-9 Gew.% auf, z.B. aufgrund Adsorption von Wasser aus der Umgebung oder Verwendung von feuchten pharmazeutisch verträglichen Hilfsstoffen.

Das in dem erfindungsgemäßen Verfahren eingesetzte Granulat, welches das Hydrochloridsalz von Donepezil in einer kristallinen und dehydratisierten Form und bevorzugt die polymorphen Formen II, III oder V, oder Gemische davon, enthält, wird durch Feuchtgranulierung erhalten.

So umfasst die Feuchtgranulierung in einer bevorzugten Ausführungsform die Schritte:
a) Herstellen einer festen Mischung, enthaltend ein Hydrochloridsalz von Donepezil und pharmazeutisch verträgliche Hilfsstoffe,
b) Granulieren der Mischung mit einer Granulierflüssigkeit bestehend aus Wasser, organischen Lösungsmitteln oder Mischungen davon, und
c) Trocknen des im Schritt (b) erhaltenen feuchten Granulats, um ein getrocknetes Granulat zu erhalten.

Zur Herstellung der festen Mischung im Schritt a) wird vorzugsweise ein Hydrochloridsalz von Donepezil in einer kristallinen Form verwendet, insbesondere ein Hydrochloridsalz von Donepezil, das in einer kristallinen und hydratisierten Form vorliegt. In einer bevorzugten Ausführungsform wird eine feste Mischung verwendet, die die polymorphe Form I oder IV, oder Gemische davon, enthält. So ist insbesondere ein Verfahren bevorzugt, das die Schritte umfasst:
a) Herstellen einer festen Mischung, enthaltende das Hydrochloridsalz von Donepezil in der polymorphen Form I und pharmazeutisch verträgliche Hilfsstoffe,
b) Granulieren der Mischung mit einer Granulierflüssigkeit bestehend aus einer Mischung von Wasser und einem Alkohol (wie etwa Ethanol oder Isopropanol),
c) Trocknen des im Schritt (b) erhaltenen feuchten Granulats, wobei das getrocknete Granulat das Hydrochloridsalz von Donepezil in der polymorphen Form V enthält,
d) Herstellen einer Tablettenmasse, umfassend das Granulat aus Schritt (c), und
e) Verpressen der Tablettenmasse zu einer Tablette, die da Hydrochloridsalz von Donepezil in amorpher Form enthält.

Es wurde gefunden, dass sich die wasserreichen, d.h. hydratisierten und kristallinen Formen des Donepezilhydrochlorids durch geeignete Wahl der Trocknungsbedingungen für das Granulat in Schritt (c) in die dehydratisierten und kristallinen Formen umwandeln lassen. Wenn z.B. in einem Wirbelschichttrockner das Granulat eine Temperatur von 40-55 °C, vorzugsweise 45-50 °C und einen Restwassergehalt von z.B. 3 Gew.-% oder weniger (Trockenverlust) aufweist, ist die Umwandlung der hydratisierten und kristallinen Form des Donepezilhydrochlorids in seine dehydratisierte Form vollständig. Bei einer Zulufttemperatur von 45-65 °C, vorzugsweise 50-60 °C und am meisten bevorzugt 50-55 °C und einer Fließrate der Trockenluft von ca. 800-ca. 2000 m³/h werden der vorstehend genannte Wassergehalt und die Temperatur für das Granulat nach etwa 20-40 min erreicht. Es sind aber auch andere Trocknungsarten wie die Vakuumtrocknung für das Granulat einsetzbar.

Nach Verpressen einer Tablettenmasse, die das vorstehend beschrieben Granulat enthält, liegt das Donepezilhydrochlorid in amorpher Form vor. Warum eine Umwandlung einer kristallinen Form des Hydrochloridsalzes von Donepezil, vorzugsweise einer kristallinen und dehydratisierten Form in seine amorphe Form während des Verpressens der Tablettenmasse auftritt ist noch nicht vollständig geklärt. Ohne an die folgende Erklärung gebunden sein zu wollen, wird die Umwandlung in die amorphe Form möglicherweise durch die bei dem Verpressen auftretenden hohen Druck oder schon während der Herstellung der Tablettenmasse durch Resorption von Wasser aus der Umgebung oder aus den zu der Tablettenmasse beigefügen Hilfsstoffen induziert. Die nach dem erfindungsgemäßen Verfahren hergestellte Tablette ist physikalisch stabil, d.h. es tritt keine Rekristallisation des amorphen Donepezilhydrochlorids auf.

Als einen weiteren Aspekt der vorliegenden Erfindung wurde gefunden, dass ein Hydrochloridsalz von Donepezil in einer kristallinen hydratisierten Form zur Herstellung eines Granulates verwendet werden kann, in welchem das Hydrochloridsalz von Donepezil in einer kristallinen und dehydratisierten Form enthalten ist, wobei das Kristalline, dehydratisierte Hydrochloridsalz von Donepezil einen Restwassergehalt von 3-7 Gew.-% aufweist.

Für die Herstellung der festen Mischung und der Tablettenmasse können die üblichen pharmazeutisch verträglichen Hilfsstoffe verwendet werden.

Als Füllstoffe können z.B. Cellulosepulver, Mannit, Calciumdiphosphat, verschiedene Stärken wie z.B. Maisstärke, mikrokristalline Cellulose, silizifizierte mikrokristalline Cellulose, Calciumcarbonat, Calciumlactat, Sorbit, Dextrose, Lactit, Lactose (wasserfrei oder als Monohydrat), etc. verwendet werden.

Als Bindemittel können z.B. Stärkekleister, Gelatine, Polyvinylpyrrolidon, Celluloseether wie z.B. Hydroxyethylcellulose, Hydroxypropylcellulose, niedrig substituierte Hydroxypropylcellulose, Hydroxypropylmethylcellulose, Methylcellulose oder Ethylcelluslo, Polymethacrelate, etc. verwendet werden.

Als Sprengmittel können Stärkederivate, Polyvinylpyrolidon, quervernetztes Polyvinylpyrrolidon, niedrig substituierte Natriumcarboxymethylcellulose, Natriumstärkeglycolat, mikrokristalline Cellulose, etc. verwendet werden.

Als Gleitmittel zur Verbesserung der Fließeigenschaften bzw. der Rieselfähigkeit können insbesondere der Tablettenmasse Magnesiumstearat, Calciumstearat, Natriumlaurylsulfat, Macrogol, hydriertes Rizinusöl, Natriumstearylfumarat, hydriertes Pflanzenöl, Talkum etc. zugesetzt werden.

### Beispiel

Es wurde eine feste Mischung aus Lactosemonohydrat Maisstärke, mikrokristalline Cellulose, silifizierte mikrokristalline Cellulose und Donepezilhydrochlorid hergestellt. Die feste Mischung enthielt außerdem Hydroxypropylcellulose als ein Bindemittel. Teilweise kann das Bindemittel auch in der Granulierflüssigkeit enthalten sein. Die feste Mischung wurde mit einer Granulierflüssigkeit, die aus Isopropanol und Wasser besteht (50 Gew.-%), in Kontakt gebracht. Nach der Granulierung wurde das erhaltene feuchte Granulat in einem Wirbelschichttrockner getrocknet bis die Temperatur im Granulat 40-47 °C und der Restwassergehalt 3 Gew.-% oder weniger (Trockenverlust) betrugen. Das getrocknete Granulat wurde gesiebt und mit silifizierter mikrokristalliner Cellulose, einer zusätzlichen Menge Maisstärke und Magnesiumstearat gemischt, um eine Tablettenmasse zu erhalten, die anschließend zu einer Tablette verpresst wurde. Die erhaltene Tablette konnte dann ggf. mit einem Film beschichtet werden.

Eine typische erfindungsgemäße Tablette enthält z.B. 5 mg Donepezilhydrochlorid, 75,5 mg Lactosemonohydrat, 42,0 mg Maisstärke, 2,8 mg mikrokristalline Cellulose, 2,8 mg Hydroxypropylcellulose, 11,2 mg silifizierte mikrokristalline Cellulose und 0,7 mg Magnesiumstearat, (140) mg. Dieser Tablettenkern kann ggf. mit einem Beschichtungsmaterial wie Opadry^{®} White beschichtet werden.

Die in diesem Beispiel angegebene Tablette enthält das Donepezilhydrochlorid in amorpher Form. Eine Rekristallisation des Wirkstoffes trat auch nach 6 Monaten Lagerung bei 40 °C und einer relativen Umgebungsfeuchtigkeit von 75 % nicht auf.

## Patentansprüche

1. Verfahren zur Herstellung einer das Hydrochloridsalz von Donepezil in amorpher Form enthaltenden Tablette, wobei das Verfahren den Schritt umfasst, Verpressen einer Tablettenmasse umfassend ein Granulat, wobei das Granulat ein Hydrochloridsalz von Donepezil in einer kristallinen und dehydratisierten Form enthält,
wobei das kristalline, dehydratisierte Hydrochloridsalz von Donepezil einen Restwassergehalt von 3- 7 Gew.% aufweist, und wobei das Granulat durch Feuchtgranulierung erhalten wird.

2. Verfahren Anspruch 1, wobei das Hydrochloridsalz von Donepezil in einer kristallinen und dehydratisierten Form aus den polymorphen Formen II, III und V, oder Gemischen davon, ausgewählt ist.

3. Verfahren nach Anspruch 1 order 2, wobei die Feuchtgranulierung die Schritte umfasst:
a) Herstellen einer festen Mischung, enthaltend ein Hydrochloridsalz von Donepezil und pharmazeutisch verträgliche Hilfsstoffe,
b) Granulieren der Mischung mit einer wässrigen Granulierflüssigkeit, und
c) Trocknen des im Schritt (b) erhaltenen feuchten Granulats, um ein getrocknetes Granulat zu erhalten.

4. Verfahren nach Anspruch 3, wobei die feste Mischung im Schritt (a) ein Hydrochloridsalz von Donepezil in einer kristallinen Form enthält.

5. Verfahren nach Anspruch 4, wobei das kristalline Hydrochloridsalz von Donepezil in einer hydratisierten Form vorliegt.

6. Verfahren nach Anspruch 5, wobei das Hydrochloridsalz von Donepezil in einer kristallinen und hydratisierten Form aus den polymorphen Formen I und IV, oder Gemischen davon, ausgewählt ist.

7. Verfahren nach Anspruch 6, umfassend die Schritte:
a) Herstellen einer festen Mischung, enthaltend das Hydrochloridsalz von Donepezil in der polymorphen Form I und pharmazeutisch verträgliche Hilfsstoffe,
b) Granulieren der Mischung mit einer wässrigen Granulierflussigkeit,
c) Trocknen des im Schritt (b) erhaltenen feuchten Granulats, wobei das getrocknete Granulat das Hydrochloridsalz von Donepezil in der polymorphen Form V enthält,
d) Herstellen einer Tablettenmasse, umfassend das Granulat aus Schritt (c), und
e) Verpressen der Tablettenmasse zu einer Tablette, die das Hydrochloridsalz von Donepezil in amorpher Form enthält.

8. Verwendung eines Hydrochloridsalzes von Donepezil in einer kristallinen und hydratisierten Form zur Herstellung eines Granulats, wobei das Granulat ein Hydrochloridsalz von Donepezil in einer kristallinen und dehydratisierten Form enthält, und
wobei das kristalline, dehydratisierte Hydrochloridsalz von Donepezil einen Restwassergehalt von 3- 7 Gew.-% aufweist.

9. Granulat, umfassend ein Hydrochloridsalz von Donepezil in einer kristallinen und dehydratisierten Form, wobei das Kristalline, dehydratisierte Hydrochloridsalz von Donepezil einen Restwassergehalt von 3 -7 Gew.-% aufweist.

10. Granulat nach Anspruch 9, wobei das Hydrochloridsalz von Donepezil in einer kristallinen und dehydratisierten Form aus den polymorphen Formen II, III und V, oder Gemischen davon, ausgewählt ist.

## Claims

1. Process for the preparation of a tablet containing the hydrochloride salt of donepezil in an amorphous form, wherein the process comprises the steps of compacting a tablet mass comprising a granulate, wherein the granulate contains a hydrochloride salt of donepezil in a crystalline and dehydrated form, wherein the crystalline, dehydrated hydrochloride salt of donepezil has a residual water content of 3-7 % by weight, and wherein the granulate is obtained by wet granulation.

2. Process according to claim 1, wherein the hydrochloride salt of donepezil in a crystalline and dehydrated form is selected from the polymorphic forms II, III and V, or mixtures thereof.

3. Process according to claim 1 or 2, wherein the wet granulation comprises the steps of:
a) Preparing a solid mixture containing a hydrochloride salt or donepezil and pharmaceutical excipients,
b) Granulation of the mixture with an aqueous granulation liquid, and
c) Drying the wet granulate obtained in step (b) to obtain a dried granulate.

4. Process according to claim 3, wherein the solid mixture in step (a) contains a hydrochloride salt of donepezil in a crystalline form.

5. Process according in claim 4, wherein the crystalline hydrochloride salt of donepezil is in a hydrated form.

6. Process according to claim 5, wherein the hydrochloride salt of donepezil in a crystalline and hydrated form is selected from the polymorphic forms I and IV, or mixtures thereof.

7. Process according to claim 6, comprising the steps of:
a) Preparing a solid mixture containing a hydrochloride salt of donepezil in the polymorphic form I and pharmaceutical excipients,
b) Granulation of the mixture with an aqueous granulation liquid,
c) Drying the wet granulate obtained in step (b), wherein the dried granulate contains the hydrochloride salt of donepezil in the polymorphic form V,
d) Preparing a tablet mass comprising the granulate obtained in step (c), and
e) Subjceting the tablet mass to compaction to obtain a tablet containing the hydrochloride salt of donepezil in an armorphous form.

8. Use of a hydrochloride salt of donepezil in a crystalline and hydrated form for the preparation of a granulate, wherein the granulate contains a hydrochloride salt of donepezil in a crystalline and dehydrated form, and wherein the crystalline, dehydrated hydrochloride salt of donepezil has a residual water content of 3-7 % by weight.

9. Granulate comprising a hydrochloride salt of donepezil in a crystalline and dehydrated form, wherein the crystalline, dehydrated hydrochloride salt of donepezil has a residual water content of 3-7 % by weight.

10. Granulate according to claim 9, wherein the hydrochloride salt of donepezil in a crystalline and dehydrated form is selected from the polymorphic forms II, III and V, or mixtures thereof.

## Revendications

1. Procédé de préparation d'un comprimé contenant du sel de chlorhydrate de donépézil sous forme amorphe, ce procédé comprenant l'étape de compression d'une masse de comprimé comprenant un granulé contenant un sel de chlorhydrate de donépézil sous une forme cristalline et déshydratée, dans lequel le sel de chlorhydrate de donépézil cristallin déshydraté présente une teneur en eau résiduelle de 3 à 7% en poids, et dans lequel le ganulé est obtenu par granulation par voie humide.

2. Procédé selon la revendication 1, dans lequel le sel de chlorhydrate de donépézil sous une forme cristalline et déshydratée est choisi parmi les formes polymorphes II, III et V ou des mélanges de celles-ci.

3. Procédé selon la revendication 1 ou 2, dans lequel la granulation par voie humide comprend les étapes suivantes :
a) préparation d'un mélange solide contenant un sel de chlorhydrate de donépézil et des excipients pharmaceutiques compatibles,
b) granulation du mélange avec un liquide de granulation aqueux, et
c) séchage du granulé humide obtenu à l'étape (b) pour obtenir un granulé sec.

4. Procédé selon la revendication 3, dans lequel le mélange solide de l'étape (a) contient un sel de chlorhydrate de donépézil sous une forme cristalline.

5. Procédé selon la revendication 4, dans lequel le sel de chlorhydrate de donépézil cristallin se présente sous une forme hydratée.

6. Procédé selon la revendication 5, dans lequel le sel de chlorhydrate de donépézil sous une forme cristalline et hydratée est choisi parmi les formes polymorphes 1 et IV ou des mélanges de celles-ci.

7. Procédé selon la revendication 6, comprenant les étapes suivantes :
a) préparation d'un mélange solide contenant le sel de chlorhydrate de donépézil sous la forme polymorphe 1 et des excipients pharmaceutiques compatibles,
b) granulation du mélange avec un liquide de granulation aqueux,
c) séchage du granule humide obtenu à l'étape (b), dans lequel le granulé sec contient le sel de chlorhydrate de donépézil sous la forme polymorphe V,
d) préparation d'une masse de comprimé comprenant le granulé de l'étape (c), et
e) compression de la masse de comprimé en un comprimé qui contient le sel de chlorhydrate de donépézil sous forme amorphe

8. Utilisation d'un sel de chlorhydrate de donépézil sous une forme cristalline et hydratée pour préparer un granulé contenant un sel de chlorhydrate de donépézil sous une forme cristalline et déshydratée, et dans lequel le sel de chlorhydrate de donépézil cristallin déshydraté présente une teneur en eau résiduelle de 3 à 7% en poids.

9. Granulé comprenant un sel de chlorhydrate de donépézil sous une forme cristalline et déshydratée, dans lequel le sel de chlorhydrate de donépézil cristallin déshydraté présente une teneur en eau résiduelle de 3 à 7% en poids.

10. Granulé selon la revendication 9, dans lequel le sel de chlorhydrate de donépézil sous une forme cristalline et déshydratée est choisi parmi les formes polymorphes II, III et V ou des mélanges de celles-ci,
